# EUROPEAN PATENT SPECIFICATION

(11) **EP 3 308 162 B1**
(45) Date of publication and mention of the grant of the patent: **02.06.2021**
(21) Application number: 16728931.3
(22) Date of filing: 09.06.2016
(51) Int. Cl.: G01N 33/50

(54) **IN VITRO METHOD FOR QUANTIFYING NANO-OBJECTS OF MAMMALIAN SKIN CELLS**
VERFAHREN ZUR IN VITRO QUANTIFIZIERUNG VON NANOOBJEKTEN VON SÄUGETIERHAUTZELLEN
PROCÉDÉ IN VITRO DE QUANTIFICATION DES NANO-OBJETS DES CELLULES MAMMIFÈRES DE LA PEAU

(30) Priority: 11.06.2015 DE 102015109345
(43) Date of publication of application: 18.04.2018
(73) Proprietor: Riethmüller, Christoph, 48147 Münster (DE)
(72) Inventor: Riethmüller, Christoph, 48147 Münster (DE)
(74) Representative: Canzler & Bergmeier Patentanwälte Partnerschaft mbB
(86) International application number: PCT/EP2016/063187
(87) International publication number: WO 2016/198535

(56) References cited:
- KATSUO MATSUMOTO ET AL: "Objective evaluation of the efficacy of daily topical applications of cosmetics bases using the hairless mouse model of atopic dermatitis", SKIN RESEARCH AND TECHNOLOGY, vol. 11, no. 3, 1 August 2005 (2005-08-01) , pages 209-217, XP055300280, DK ISSN: 0909-752X, DOI: 10.1111/j.1600-0846.2005.00106.x
- GOERGE C GORZELANNY ET AL: "Atomic force microscopy as an innovative tool for nanoanalysis of native stratum corneum Introduction", EXP DERMATOL JOURNAL, vol. 15, 30 March 2006 (2006-03-30), pages 387-391, XP055300285,
- CHRISTIAN RANKL ET AL: "Detection of corneodesmosin on the surface of stratum corneum using atomic force microscopy", EXPERIMENTAL DERMATOLOGY, vol. 19, no. 11, 21 October 2010 (2010-10-21), pages 1014-1019, XP055300288, COPENHAGEN; DK ISSN: 0906-6705, DOI: 10.1111/j.1600-0625.2010.01179.x

## Description

Integrity of the skin barrier is a prerequisite for health. The skin barrier protects the organism against exogenous stressors and simultaneously prevents water loss.

However, uncommon skin conditions such as atopic dermatitis (AD) are increasing throughout the industrialized world without a clear knowledge about the exact cause and mechanisms. In many skin disorders including AD, the barrier function is compromised. The skin barrier is at dynamic equilibrium due to a proliferating epidermis balanced by permanent abrasion of cornified epithelial cells. Hence, corneocytes can be regarded as messengers from inside the body and minute changes of their morphology might hint at out-of-balance processes underneath.

Corneocyte ultrastructure has previously been revealed by electron microscopy, see Dawber, R. P., R. Marks, et al. (1972), "Scanning electron microscopy of the stratum corneum", Br J Dermatol 86(3): 272-281. Individual appearances have been described, most prominently the so-called "rough corneocytes"(, where the subcellular texture is not smooth, but decorated with multiple small protrusions, see Fredonnet, J., G. Gasc, et al. (2014), "Topographical and nano-mechanical characterization of native corneocytes using atomic force microscopy", J Dermatol Sci 75(1): 63-65. These nanoscale cell structures have also been termed 'bead-like' elevations (see Rankl, C., R. Zhu, et al. (2010), "Detection of corneodesmosin on the surface of stratum corneum using atomic force microscopy", Exp Dermatol 19(11): 1014-1019) and have been suggested to be associated with the age of the patient, body location and depth of the investigated layer of the stratum corneum (SC), see Gorzelanny, C., T. Goerge, et al. (2006), "Atomic force microscopy as an innovative tool for nanoanalysis of native stratum corneum", Exp Dermatol 15(5): 387-391. In said article, exemplary AFM-recordings of human stratum corneum including protrusions of two individuals (of different age) were presented. Visual inspection revealed their height profiles along arbitrary sections.

In the article "Objective evaluation of the efficacy of daily topical applications of cosmetics bases using the hairless mouse model of atopic dermatitis" by Katsuo Matsumoto et al. (2005), Skin Research and Technology, vol. 11, no. 3: 209-217, a study on mouse corneocytes has been disclosed. The authors observed vili-like-protrusions (VLP) in electron microscopic images. A classification of higher or lower VLP-density was performed by visual inspection only.

Small protrusions have been identified by others also in plantar and cheek corneocytes, see Naoe, Y., T. Hata, et al. (2010), "Bidimensional analysis of desmoglein 1 distribution on the outermost corneocytes provides the structural and functional information of the stratum corneum", J Dermatol Sci 57(3): 192-198. Since no clear biological function could be assigned yet, it is preferred here to use a phenotypic term by referring to these objects as "circular nano-objects" (CNOs). Importantly, the CNOs appear to be missing in healthy skin from the forearm. An irregular surface pattern has also been described in other skin diseases like ichthyosis vulgaris and psoriasis. In a recent study the presence of CNOs has been suggested to correlate with the impairment of the skin barrier function, see Naoko, O., H. Satoshi, et al. (2013), "Changes in villus-like projections of corneocytes from the facial skin in normal infants with or without infantile eczema; useful parameter to assess barrier function", Skin Res Technol 19(4): 361-367.

A standard non-invasive technique to obtain samples from the human stratum corneum is the tape stripping procedure, see Lademann, J., U. Jacobi, et al. (2009), "The tape stripping procedure - evaluation of some critical parameters", Eur J Pharm Biopharm 72(2): 317-323. Combined with chemical analysis methods, it serves in pharmacokinetics for retrieving skin-permeating compounds or to determine the endogenous content of natural moisturising factor (NMF). Likewise, the adhering corneocytes may be subjected to all kinds of high-resolution microscopy with or without labelling. Various methods of analysing corneocytes on tape strips are subject of patented methods, but they either focus on cell size, cell density, etc., or they use staining / labelling procedures (EP 1 845 359 A2).

Atomic force microscopy (AFM) became a versatile tool for biological studies on single biomolecules, viruses or cells. Since no sample processing is necessary, investigation of biological specimen close to physiological conditions is possible with only a minimum of procedure-derived artefacts. Corneocytes have already been investigated by AFM and CNOs have already been observed, see above the articles by Gorzelanny, C. et al. and Fredonnet, J. et al. However, no relevant conclusions have been drawn from these observations regarding any diagnosis.

It is the technical problem of the present invention to provide a method for extracting relevant diagnostic information from observations of CNOs on corneocytes of mammalian skin cells.

This technical problem is solved by an in vitro method for quantifying nano-objects of mammalian skin cells according to claim 1, comprising the following steps:
a) Collecting mammalian corneocytes;
b) Analysing the surface topography of corneocytes at nanoscale resolution;
c) Identifying subcellular objects of typical size (perimeter and/or height) smaller than 500 nm with a circularity index > 0.5 (circular nano-objects, CNOs) and having an area less than 1 µm²;
d) Counting these CNOs;
e) Relating the number of CNOs to a unit area for obtaining a density parameter, especially a structural index in the form of a Dermal Topographic Index, DTI.

The invention is also directed to the use of said in vitro method for various assessments and tests, e.g., for assessing an impaired skin barrier function of an individual, for assessing an elevated inflammatory condition, for giving or determining an individual risk factor for the future development of a skin disease, and/or for allergy testing, including food allergies. Likewise, the method may be used for testing the efficacy or side effects of drugs, including topically and systemically applied drugs, for testing skin irritants, e.g., to exposures at workspaces, and/or for testing the effect of cosmetic formulations, creams, emollients, sun blockers, and skin application agents in general.

Advantageous embodiments of the invention are described in the subclaims.

It is to be noted that analysing the surface topography of corneocytes at nanoscale resolution may be done by various methods, among which are: atomic force microscopy (AFM); scanning electron microscopy (SEM); digital holographic microscopy (DHM); (confocal) Raman microscopy; confocal reflectance microscopy; (confocal) fluorescence microscopy; and optical interferometry.

According to one preferred embodiment of the invention, a visualization of the CNOs - which here are defined as subcellular objects of typical size smaller than 500 nm (perimeter and/or height) with a circularity index > 0.5 - may be accomplished by specific labelling and subsequent label-based microscopy techniques, e.g. fluorescence microscopy, or immune-gold SEM or SEM-EDX. One specific respective embodiment regarding the labelling involves antibodies to biochemical constituents of the CNOs for counting these.

It is preferred that the analysed area is less than the surface of one corneocyte. Recording many randomly accessed areas is advantageous as this makes it possible to obtain a representative value by averaging the analysis results to cope with biological heterogeneity.

Thus, the invention allows a linking of nanostructure count to skin diagnostics, which has not been known so far. Specifically, the invention provides a possibility to quantify circular nano-objects (CNOs) on corneocytes to yield an in vitro diagnostic parameter for patient skin.

The method of the invention may be applied to different anatomical regions of the volunteer or patient to set up an individual map of CNO densities across the body.

For the identification of CNOs a neural network within an automatic may be used. For the skilled person in the art it is clear that other known algorithms may be applicable for the evaluation of the data.

The determination of the CNOs takes place at a part of the cellular surface, wherein the subdivided part of the cell or cell surface comprises a length of preferably 5 to 50 µm. With respect to the counting of CNOs it is preferred when the subdivided part of the cell comprises a deviational volume in the range of 0.2 to 20 µm in xy-axis and < 500 nm in z-axis. The local deviational volume is defined as in EP 2 435 829 B1, i.e., as a nanoscale excursion in z-direction over a predefined mask in xy-plane, wherein the local protruding, positive or depressed, negative volume as compared to the mean surface level of said predefined mask is evaluated (see claim 1 of EP 2 435 829 B1).

It is preferred if the number of CNOs per unit area constitute an index number for simple comparison, the so-called Dermal Topographical Index (DTI). For this purpose, the data of a calibrated or standard sample are obtained preferably by pooling samples' data of healthy volunteers. The healthy groups may be divided into subclasses along classical dermatological qualifiers, as for instance the subjects' age, sex, skin pigmentation, ethnical groups, etc.

According to one embodiment of the invention, additional nanoscale structures like non-circular objects (circularity index < 0.5) or ridges or fibers or compositions thereof may be used for detailed sub-classification of the index.

Preferably, the method according to the invention is used to produce an in vitro diagnostic marker for skin condition.

The method according to the invention is preferably used for the detection of a morphometric marker related to diseases, wherein the diseases are chosen from the group of immune, allergic, atopic, tumorous, or other skin diseases. A person skilled in the art will recognize that the present invention is not limited to the listed diseases but also applicable to any disease that involves skin malfunction.

The method according to the invention is preferably intended for determining corneocyte surface structures as an in vitro diagnostic marker in the prophylaxis, diagnosis, therapy, follow-up and/or aftercare of a therapy in any of the diseases mentioned above. Besides this the method is suitable and intended for determining cellular mechanical or contractile forces.

The method according to the invention may be used in the production or screening of a drug for the treatment of any of the diseases mentioned above comprising pharmaceutical compositions, antibodies, proteins, peptides, nucleic acids or chemicals, but is not limited to this substances.

The method according to the invention may also be used for a cell-culture based system of diagnosis. Specific patterns of topographical structures will be related to skin disease induced alteration of the cell and tissue function. Additionally, it is possible to determine the local extension of a disease if cell samples from different regions of the body are used for the method according to the invention.

The present invention provides a method to determine the number of CNOs irrespective of their biochemical characterisation. There are indications which hint that the CNOs are corneodesmosomes. The present inventor also assumes that f-actin or keratin-enforced anchoring scaffolds function as cell-cell attachment sites. However, at the moment it is sufficient to know that the density of CNOs is defined as Dermal Topographical Index (DTI), which gives a simple scalar value to be used for classification of the skin tissue status. The method according to the invention is based on the observation that the surface texture changes within a nanometer range in the z-direction (height), when cells are growing differentiating or are being stressed. Corneocytes are used as reporters from inside the body to integrate cellular stress information of the past few weeks within the individual under investigation.

Different types of skin disorder lead to distinct alterations in DTI. Some examples are:
- Atopic Dermatitis (AD), a common inflammatory skin condition;
- Actinic Keratosis, a pre-stage of non-melanoma skin cancer;
- Contact Dermatitis (reaction to chemicals or other sensitizing agents);
- Proriasis;
- Ichtyosis (a non-inflammatory skin disease);
- Dry skin;
- Sensitive skin.

When quantitated in various experiments, the above listed skin conditions exhibited an elevated number of CNOs, i.e., a higher DTI value. The advantage of this method is that it delivers information about tissue status without the need for a biopsy. Moreover, it circumvents the problems of optical microscopy on tape strips that arise from turbidity and autofluorescence of the tape material.

As alternatives to adhesive tape stripping, the following methods may be employed: skin biopsies; dandruff shedding; cyanacrylate ruptures; and cultivation of skin cells in vitro (keratinocytes).

The invention will be further described by figures and examples without being limited to the described embodiments.

### Sample collecting

The skin was sampled using a standard method. Clinically unaffected skin on the subjects' volar forearm was used for stratum corneum sampling. Circular adhesive tape strips (3.8 cm², D-Squame, Monaderm, Monaco, France) were attached to the forearm skin and pressed gently for 10 seconds. Ten consecutive tape strips were sampled from the same site. Nine tape strips were discarded and the tenth tape strip was gently removed, placed in a closed vial and stored at room temperature until analysis.

### AFM

Corneocytes were analysed by atomic force microscopy (AFM) as described before, see the article by Gorzelanny, C. et al. Briefly, in each case the 10^{th} tape strip was subjected to AFM measurements carried out using a Multimode AFM equipped with a Nanoscope III controller and software version 5.30sr3 (Digital Instruments, Santa Barbara, CA, USA). Silicon-nitride tips on V-shaped gold-coated cantilevers were used (0.01 N/m, MLCT, VEECO, Mannheim, Germany). Imaging was performed in air at ambient temperature with forces less than 1 nN at 1-3 scan lines per second (1-3 Hz) with 512*512 pixels resolution. For nano-object analysis, subcellular scan areas of (20 µm)² were recorded.

Topography of the cell surfaces was analysed using the nAnostic™-method applying custom-built, proprietary algorithms (Serend-ip GmbH, Münster, Germany) according to previously granted US 8,798,935 B2 and EP 2 435 829 B1. Briefly, each nanostructure protruding from the mean surface level is morphometrically evaluated. These measurements are then filtered by size and shape through computer vision. Here, only structures (namely, CNOs being defined as subcellular objects of typical perimeter and/or height smaller than 500 nm with a circularity index > 0.5) of positive local deviational volume (LDV) smaller than 500 nm in height and an area of less than 1 µm² were considered. The object count is given per (20 µm)² unit area. From each sample, 10 randomly accessed regions were recorded, evaluated and averaged to yield one index number.

The following clinical and subclinical examples 1 - 4 were performed by using the method according to the invention. The Figures 1-3 refer to the more detailed description of Example 1 (atopic dermatitis), wherein:
- Figure 1A-B: depicts nanostructures on human corneocytes;
- Figure 2A-D: depicts computer vision images; and
- Figure 3A-C: depicts the effect of test persons' age, pigmentation or disease state.

### 1. Atopic dermatitis

Atopic dermatitis (AD; or atopic eczema) is an inflammatory skin condition, which is highly common in the industrialized world.

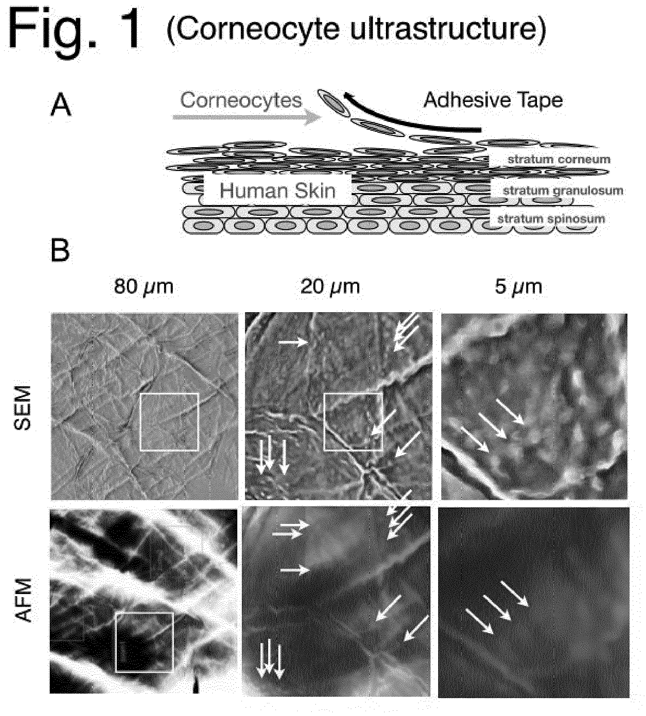

Corneocytes of the volar forearm of volunteers with or without AD were sampled by the tape-stripping procedure as depicted in Fig. 1A. On SEM-recordings (Fig. 1B, upper row; 80 µm), corneocytes appear as flat sheets with characteristic ridges and small furrows at the border between neighboring corneocytes. A zoom in to 20 µm and 5 µm reveals circular nano-objects (marked by arrows) often near the ridges as have already been described by others before, still lacking a clear identification. To further characterize these structures by their true height, AFM was performed on the same sample (Fig. 1B, lower row). It was possible to image identical regions through both methods as can be recognized by a triangle of ridges. The same characteristic morphology is exhibited by SEM and AFM. The circular structures are recognizable as well (arrows), albeit less clearly. The corneocyte surfaces were very flat, height differences are usually below 1 µm (in z-direction). Basically all the morphology appears similar in AFM and SEM, so neither method produces considerable artifacts. Here, we focus on the circular spots (depicted by arrows), which were named "circular nano objects" (CNOs).

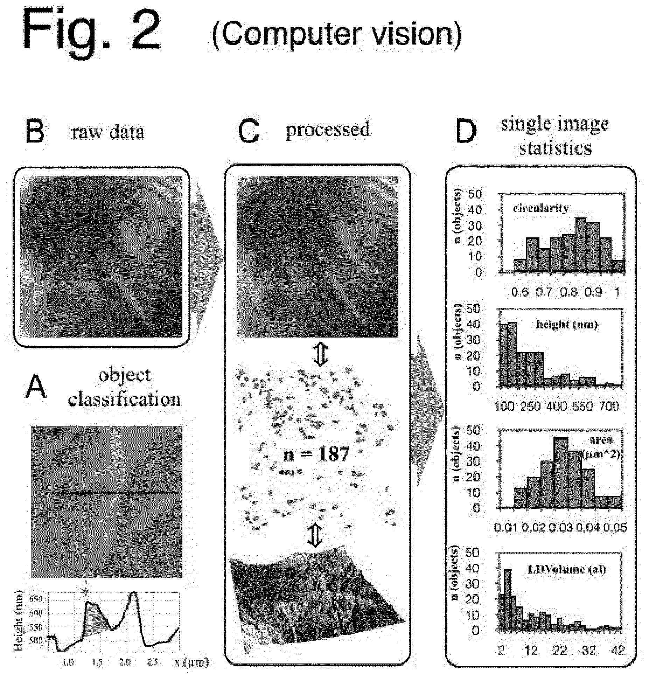

Computer vision on AFM-images was applied here. A basic scheme of the procedure is depicted in Fig. 2. A subcellular "object of interest" (circular nano-object) is defined (manually) by its protrusion or elevation above mean surface level, here defined by 100 nm < h < 750 nm, see the green area under the profile curve of Fig. 2A. The in-plane-shape (round or fiber-like) assists in further classification. From a typical raw image of (20 µm)² (Fig. 2B) many "objects of interest" were identified by a trained software tool (Fig. 2C). All three representations (2D, object mask and 3D) contained identical information about the green objects, which were n=187 in this particular example. Morphometry can be performed easily to show distributions of measures like circularity, area, height and size (Fig. 2D). The total size was here calculated as the local deviational volume (LDV) as described in US-Pat 8,798,935 B2 and EP 2 435 829 B1, enumerating typically to 11.3 al (atto-liters, 10⁻¹⁸ liters). The LDV approximates the difference between the true surface and a - hypothetical - smooth surface.

The automation allowed to analyze a sufficient amount of data in an operator-independent manner and delivered robust results. It is to be noted that the object count was independent of the AFM-instrument manufacturer. The physical repeatability was high: deviation in object count was less than 10% through ten consecutive scans over the same area - including artifacts like scan lines or instrument drift etc. (data not shown). Overall, the recording and evaluation process is objective, stable and sensitive.

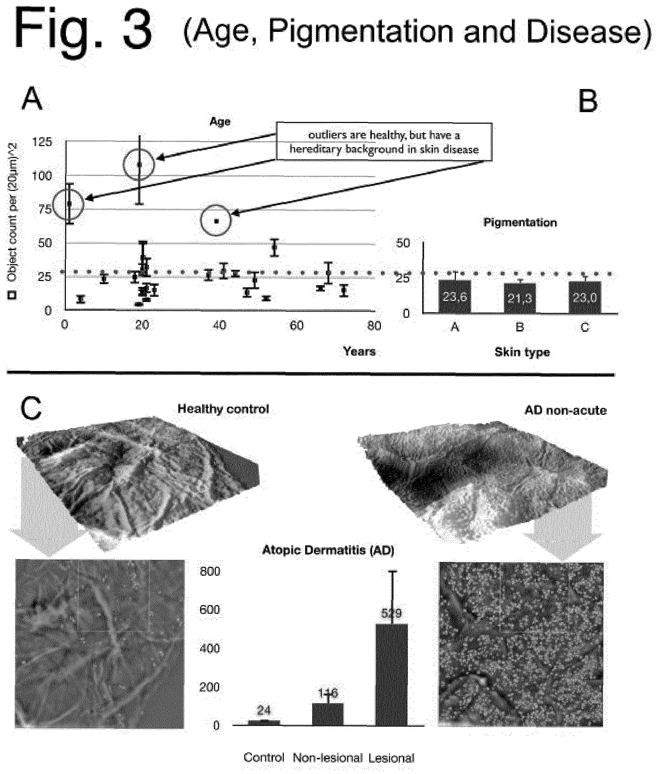

To test for the physiological relevance of the nano-objects, the test cohort was classified following obvious macroscopic criteria: age, pigmentation and disease (Fig. 3). Since an earlier AFM-report has assigned their appearance onto the age of subjects (see the article by Gorzelanny, C. et al.), this was performed first. Tape strips were collected of 21 healthy individuals that never had experienced any skin disease and subjected to the nano-object counting procedure (Fig. 3A). Samples were analyzed as described for Fig. 2. DTI values were between 8.1 and 108.9. No correlation with age was detected. Three individuals with a higher score turned out to have relatives with a positive history of psoriasis or AD - hinting at a genetic contribution to corneocyte ultrastructure. Disregarding these outlier values yielded a healthy (normal) DTI value of 27 ± 6.3.

To see whether a second dermatological criterion has any influence, the test cohort was grouped according to their pigmentation types A-D according to Fitzpatrick (Fig. 3B). No influence of pigmentation could be observed.

As a third obvious macroscopic criterion, skin disease was investigated whether it would affect corneocyte ultrastructure (Fig. 3C). Samples from five volunteers with atopic dermatitis (AD) were taken from sites of visible inflammation (lesional sites). There was abundant occurrence of nano-objects (CNOs), namely (529 ± 277 SD) per image. Moreover, at regions without any visible sign of inflammation (non-lesional sites), there still was a clearly elevated density of CNOs (116 ± 53 SD) as compared to healthy subjects (24.0 ± 21.1 SD). P-values of the students t-test are 0.0000127 for control group versus non-lesional and 0.0000424 for non-lesional versus lesional sites. After 6 weeks of classical corticosteroid therapy in AD patients, the DTI was found to have decreased by 35%. Accordingly, the invention can also be used to monitor the effect of a therapy.

### 2. Psoriasis

Psoriasis is the cutaneous manifestation of a systemic disease of presumably autoimmune origin. One characteristic of Psoriasis is an accelerated turnover of keratinocytes at inflammatory plaques that show a preference for (but not being limited to) regions of thicker epidermis as on the elbow or knee - in contrast to atopic dermatitis, which is rather found where stratum corneum is thinner (inner side of forearm, face, neck etc.). Disease appearance and progression of psoriasis show an individually broad variation.

Volunteers that were diagnosed psoriasis were sampled on lesional and non-lesional sites. While the lesional sites exhibited close to maximal DTI values of around 500, the non-lesional sites were still significantly elevated at DTI-values of above 100. Hence, also a psoriatic skin can be characterized by the method of the invention even at clinically unaffected sites.

### 3. Actinic keratosis (AK)

Actinic keratosis is characterized by proliferation of keratinocytes, which is a consequence of UV-light exposure. It is controllable but may develop into a squamous cell carcinoma. Therefore, it is regarded as a "precancerosis". AK has just been accepted by German healthcare officials as an occupational disease.

A volunteer diagnosed actinic keratosis was sampled on 1) a lesional region on the forearm, 2) a non-lesional region in close proximity (light-exposed area), and 3) a non-lesional region from a non-light exposed region (inner side of the arm close to the armpit). The DTI values of 1) 400, 2) 300, and 3) 100 demonstrate a clearly elevated value at non-lesional sites. This renders the method of the invention a toll for the early detection of UV-induced skin cancer.

### 4. Exposition to detergents

Contact dermatitis is an inflammatory reaction after exposure to detergents or other irritant compounds or materials. It is a socioeconomic problem for employees being exposed to chemicals at their workplace. In an experiment, healthy volunteers were exposed to a standard detergent solution of 0.1 % sodium dodecyl sulphate (SDS) for 24h.

Before the exposition to detergent, the volunteer's DTI was as low as 40. After three days of SDS exposure, the many circular nano-objects CNOs arose such that the DTI increased to a value of 370, which is already half maximal. Hence, a toxic influence by detergents or other noxious conditions can be indicated by DTI.

The above experiments demonstrate that the invention provides a method for quantifying the nanoscale texture of corneocytes, which are subject to change in diseased states. Using the method according to the invention, it was for the first time possible to demonstrate that nanostructures on skin cells indicate a compromised skin condition.

## Claims

1. In vitro method for quantifying nano-objects of mammalian skin cells comprising the following steps:
a) Providing mammalian corneocytes;
b) Analysing the surface topography of corneocytes at nanoscale resolution;
c) Identifying subcellular objects of height smaller than 500 nm with a circularity index > 0.5 (circular nano-objects, CNOs) and having an area less than 1 µm²;
d) Counting these CNOs by an automated software routine;
e) Relating the number of CNOs to a unit area for obtaining a density parameter, especially a structural index in the form of a dermal topographic Index, DTI.

2. Method according to claim 1, **characterized in that** the corneocytes according to step a) of claim 1 have been collected according to one of the following methods:
a) adhesive tape stripping;
b) skin biopsies;
c) dandruff shedding;
d) cyanacrylate ruptures;
e) cultivation of skin cells in vitro (keratinocytes).

3. Method according to claim 1 or 2, **characterized in that** the nano-objects according to step b) of claim 1 are analysed by one of the following methods:
a) atomic force microscopy (AFM);
b) scanning electron microscopy (SEM);
c) digital holographic microscopy (DHM);
d) Raman microscopy;
e) confocal reflectance microscopy;
f) fluorescence microscopy;
g) optical interferometry.

4. Method according to any one of the preceding claims, **characterized in that** also surface structures with a circularity index < 0.5 are counted to assist and/or complement the CNO count.

5. Use of the in vitro method according to any one of preceding method claims for assessing an impaired skin barrier function of an individual, or for assessing an elevated inflammatory condition.

6. Use of the in vitro method according to any one of preceding method claims as a diagnostic marker method for skin diseases.

7. Use of the in vitro method according to any one of preceding method claims:
a) for giving or determining an individual risk factor for the future development of a skin disease;
b) for allergy testing, preferably selected from skin diseases and food allergies;
c) for testing the efficacy or side effects of drugs, including topically and systemically applied drugs;
d) for testing skin irritants, e.g., to exposures at workspaces;
e) for testing the effect of cosmetic formulations, creams, emollients, sun blockers, and skin application agents in general.

## Patentansprüche

1. In-vitro-Verfahren zur Quantifizierung von Nanoobjekten von Hautzellen von Säugetieren umfassend die folgenden Schritte:
a) Gewinnen von Korneozyten von Säugetieren;
b) Analysieren der Oberflächen-Topographie der Korneozyten in nanoskaliger Auflösung;
c) Identifizieren subzellulärer Objekte einer Höhe kleiner als 500 nm mit einem Zirkularitätsindex > 0,5 (zirkuläre Nanoobjekte, CNOs) und die eine Fläche kleiner als 1 µm² aufweisen;
d) Zählen dieser CNOs mittels einer automatischen Software-Routine;
e) Beziehen der Anzahl an CNOs auf eine Flächeneinheit, um einen Dichteparameter zu erhalten, insbesondere einen strukturellen Index in Form eines dermalen topographischen Index (DTI).

2. Verfahren nach Anspruch 1, **dadurch gekennzeichnet, dass** die Korneozyten gemäß Schritt a) gemäß Anspruch 1 durch eine der folgenden Verfahren gewonnen wurden:
a) Abziehen eines Klebestreifens;
b) Hautbiopsien;
c) Hautschuppenablösung;
d) Cyanacrylat-Abrisse;
e) Kultivierung von Hautzellen in vitro (Keratinozyten).

3. Verfahren nach Anspruch 1 oder 2, **dadurch gekennzeichnet, dass** die Nanoobjekte gemäß Schritt b) gemäß Anspruch 1 durch eine der folgenden Techniken analysiert werden:
a) Rasterkraftmikroskopie (atomic force microscopy, AFM);
b) Rasterelektronenmikroskopie (scanning electron microscopy, SEM);
c) Digitale Holographie-Mikroskopie (digital holographic microscopy, DHM);
d) Raman Mikroskopie;
e) Konfokale Reflexionsmikroskopie;
f) Fluoreszenz-Mikroskopie;
g) Optische Interferometrie.

4. Verfahren nach einem der vorhergehenden Ansprüche, **dadurch gekennzeichnet, dass** auch Oberflächenstrukturen mit einem Zirkularitätsindex von < 0,5 gezählt werden, um die Zählung der CNOs zu unterstützen und/oder zu komplementieren.

5. Verwendung des In-vitro-Verfahrens nach einem der vorhergehenden Verfahrensansprüche zur Bestimmung einer gestörten Hautbarrierefunktion eines Individuums oder zur Bestimmung eines erhöhten entzündlichen Zustandes.

6. Verwendung des In-vitro-Verfahrens nach einem der vorhergehenden Verfahrensansprüche als diagnostisches Marker-Verfahren für Hauterkrankungen.

7. Verwendung des In-vitro-Verfahrens nach einem der vorhergehenden Verfahrensansprüche:
a) zur Benennung oder Bestimmung eines individuellen Risikofaktors für die zukünftige Entstehung einer Hauterkrankung;
b) zur Allergietestung, vorzugsweise ausgewählt aus Hautkrankheiten sowie Nahrungsmittelallergien;
c) zur Bestimmung der Wirksamkeit oder der Nebenwirkungen von Wirkstoffen, einschließlich topisch sowie systemisch angewandter Wirkstoffe;
d) zur Testung von hautreizenden Stoffen, z.B. bei Expositionen am Arbeitsplatz.
e) zum Testen der Wirkung von kosmetischen Formulierungen, Cremes, Emulsionen, Sonnenschutzmitteln sowie Hautanwendungsmitteln im Allgemeinen.

## Revendications

1. Procédé in vitro pour quantifier les nano-objets des cellules de la peau de mammifères comprenant les étapes suivantes :
a) Fournir des cornéocytes de mammifères ;
b) Analyser la topographie de surface des cornéocytes à une résolution nanométrique ;
c) Identifier des objets sous-cellulaires d'une hauteur inférieure à 500 nm avec un indice de circularité > 0,5 (nano-objets circulaires ; CNO) et ayant une surface inférieure à 1 µm² ;
d) Compter ces CNOs par une routine logicielle automatisée ;
e) Relier le nombre de CNOs à une unité de surface pour obtenir un paramètre de densité, en particulier un indice structurel sous la forme d'un indice topographique dermique (DTI).

2. Procédé selon la revendication 1, **caractérisé en ce que** les cornéocytes selon étape a) de la revendication 1 ont été collectés selon l'une des méthodes suivantes :
a) dénudage de bande adhésive ;
b) biopsies de la peau ;
c) mue de pellicules ;
d) ruptures de cyanacrylate ;
e) culture de cellules de peau in vitro (keratinocytes).

3. Procédé selon la revendication 1 ou 2, **caractérisée en ce que** les nano-objets selon étape b) de la revendication 1 sont analysés par l'une des méthodes suivantes :
a) microscopie à force atomique (AFM) ;
b) microscopie électronique à balayage SEM) ;
c) microscopie holographique numérique (DHM) ;
d) microscopie Raman ;
e) microscopie confocale à réflectance ;
f) microscopie à fluorescence ;
g) interférométrie optique.

4. Procédé selon l'une quelconque des revendications précédentes, **caractérisée en ce que** des structures de surface d'un indice de circularité < 0,5 sont également comptées pour assister et/ou compléter le comptage des CNOs.

5. Utilisation de la procédé in vitro selon l'une quelconque des revendications précédentes pour la détermination d'une barrière de peau déréglé ou d'un état inflammatoire accru d'une personne.

6. Utilisation de la procédé in vitro selon l'une quelconque des revendications précédentes comme méthode de marquage diagnostique pour les maladies de la peau.

7. Utilisation de la procédé in vitro selon l'une quelconque des revendications précédentes :
a) pour donner ou pour déterminer un facteur de risque individuel pour le développement futur d'une maladie de la peau ;
b) pour tester les allergies, de préférence choisies parmi les maladies de la peau et les allergies alimentaires ;
c) pour tester l'efficacité ou les effets secondaires des médicaments, y compris les médicaments à application topiques et systématique ;
d) pour tester les irritants cutanés, par exemple, aux expositions sur les lieux de travail ;
e) pour tester les effets de formulations cosmétiques, de crèmes, d'émulsions, d'agents de protection solaire et d'agents d'application sur la peau en général.
